# EUROPEAN PATENT SPECIFICATION

(11) **EP 3 998 947 B1**
(45) Date of publication and mention of the grant of the patent: **02.09.2026**
(21) Application number: 20840867.4
(22) Date of filing: 13.07.2020
(51) Int. Cl.: A61B 5/24, A61N 1/36, A61B 5/00

(54) **MONITORING A QUALITY OF NEURAL RECORDINGS**
ÜBERWACHUNG DER QUALITÄT NEURONALER AUFZEICHNUNGEN
SURVEILLANCE D'UNE QUALITÉ D'ENREGISTREMENTS NEURONAUX

(30) Priority: 12.07.2019 AU 2019902485
(43) Date of publication of application: 25.05.2022
(73) Proprietor: Saluda Medical Pty Ltd, Macquarie Park NSW 2113 (AU)
(72) Inventor: PARKER, Daniel, Artarmon, New South Wales 2064 (AU); OBRADOVIC, Milan, Artarmon, New South Wales 2064 (AU); KARANTONIS, Dean, Artarmon, New South Wales 2064 (AU); GUELTON, Ivan, Artarmon, New South Wales 2064 (AU); ASCONE, Stephanie, Artarmon, New South Wales 2064 (AU); NARAYANAN, Michael, Artarmon, New South Wales 2064 (AU)
(74) Representative: Abel & Imray LLP
(86) International application number: PCT/AU2020/050725
(87) International publication number: WO 2021/007615

(56) References cited:
- WO-A1-2013/116161
- WO-A1-2015/074121
- WO-A1-2020/124135
- US-A1- 2006 287 609
- US-A1- 2018 228 391
- US-B2- 9 044 155
- GERT VAN DIJCK ET AL: "Toward Automated Electrode Selection in the Electronic Depth Control Strategy for Multi-unit Recordings", 22 November 2010, SAT 2015 18TH INTERNATIONAL CONFERENCE, AUSTIN, TX, USA, SEPTEMBER 24-27, 2015; [LECTURE NOTES IN COMPUTER SCIENCE; LECT.NOTES COMPUTER], SPRINGER, BERLIN, HEIDELBERG, PAGE(S) 17 - 25, ISBN: 978-3-540-74549-5, XP019158600
- GERT VAN DIJCK ET AL: "Review of machine learning and signal processing techniques for automated electrode selection in high-density microelectrode arrays", BIOMEDIZINISCHE TECHNIK, vol. 59, no. 4, 15 November 2013 (2013-11-15), DE, XP055750043, ISSN: 0013-5585, DOI: 10.1515/bmt-2013-0037

## Description

### Technical Field

The present invention relates to electrical recording of neural activity such as compound action potentials evoked by neurostimulation, and in particular to systems for improved detection of neural responses in a recording when the recording is obtained in the presence of stimulus artefact, noise and the like.

### Background of the Invention

Electrical neuromodulation is used or envisaged for use to treat a variety of disorders including chronic pain, Parkinson's disease, and migraine, and to restore function such as hearing function and motor function. A neuromodulation system applies an electrical pulse to neural tissue in order to generate a therapeutic effect. Such a system typically comprises an implanted electrical pulse generator, and a power source such as a battery that may be rechargeable by transcutaneous inductive transfer. An electrode array is connected to the pulse generator, and is positioned close to the neural pathway(s) of interest. An electrical pulse applied to the neural tissue by an electrode causes the depolarisation of neurons, which generates propagating action potentials whether antidromic, orthodromic, or both, to achieve the therapeutic effect.

When used to relieve chronic pain for example, the electrical pulse is applied to the dorsal column (DC) of the spinal cord and the electrode array is positioned in the dorsal epidural space. The dorsal column fibres being repeatedly stimulated in this way inhibit the transmission of pain from that segment in the spinal cord to the brain.

In general, the electrical stimulus generated in a neuromodulation system triggers a neural action potential which then has either an inhibitory or excitatory effect. Inhibitory effects can be used to modulate an undesired process such as the transmission of pain, or excitatory effects can be used to cause a desired effect such as the contraction of a muscle or stimulation of the auditory nerve.

The action potentials generated among a large number of fibres sum to form a compound action potential (CAP). The CAP is the sum of responses from a large number of single fibre action potentials. When a CAP is electrically recorded, the measurement comprises the result of a large number of different fibres depolarising. The propagation velocity is determined largely by the fibre diameter and for large myelinated fibres as found in the dorsal root entry zone (DREZ) and nearby dorsal column the velocity can be over 60 ms⁻¹. The CAP generated from the firing of a group of similar fibres is measured as a positive peak P1 in the recorded potential, then a negative peak N1, followed by a second positive peak P2. This is caused by the region of activation passing the recording electrode(s) as the action potentials propagate along the individual fibres, producing the typical three-peaked response profile. Depending on stimulus polarity and the recording electrode(s) configuration, the measured profile of some CAPs may be of reversed polarity, with two negative peaks and one positive peak.

To better understand the effects of neuromodulation and/or other neural stimuli, and for example to provide a stimulator controlled by neural response feedback, it is desirable to accurately detect and record a CAP evoked by the stimulus. Evoked CAPs (ECAPs) are less difficult to detect when they appear later in time than the artefact, or when the signal-to-noise ratio is sufficiently high. The artefact is often restricted to a time of 1 - 2 ms after the stimulus and so, provided the neural response is detected after this time window, a response measurement can be more easily obtained. This is the case in surgical monitoring where there are large distances (e.g. more than 12 cm for nerves conducting at 60 ms⁻¹) between the stimulating and recording electrodes so that the propagation time from the stimulus site to the recording electrodes exceeds 2 ms.

However, to characterize the responses from the dorsal columns, high stimulation currents and close proximity between electrodes are required. Similarly, any implanted neuromodulation device will necessarily be of compact size, so that for such devices to monitor the effect of applied stimuli the stimulus electrode(s) and recording electrode(s) will necessarily be in close proximity. In such situations the measurement process must overcome artefact directly. However, this can be a difficult task as an observed ECAP signal component in the neural measurement will typically have a maximum amplitude in the range of microvolts. In contrast a stimulus applied to evoke the ECAP is typically several volts and results in electrode artefact, which manifests in the neural measurement as a decaying output of several millivolts partly or wholly contemporaneously with the ECAP signal, presenting a significant obstacle to isolating or even detecting the much smaller ECAP signal of interest.

The difficulty of this problem is further exacerbated when attempting to implement CAP detection in an implanted device. Typical implants have a power budget which permits a limited number, for example in the hundreds or low thousands, of processor instructions per stimulus, in order to maintain a desired battery lifetime. Accordingly, if a CAP detector for an implanted device is to be used regularly (e.g. of the order of once a second), then care must be taken that the detector should consume only a small fraction of the power budget.

A further complexity arises from the increasing configurability of stimulation modes and recording modes of neurostimulation devices. Variables include selection of stimulation electrodes and/or recording electrodes from a potentially large number of available electrodes upon an implanted electrode array, multiple stimulation parameters, and multiple recording parameters. Clinical verification of suitable operation of a neurostimulation device ideally should include identifying the optimal settings for such variables for optimal therapeutic efficacy, however the number of combinations which must be tested can be very large and at present must largely be carried out by a clinician, making the clinical fitting process time consuming and expensive.

US2018228391 discloses a method for processing a neural measurement obtained in the presence of noise, in order to detect whether a locally evoked neural response is present in the neural measurement. A first neural measurement is obtained from a first sense electrode. A second neural measurement is contemporaneously obtained from a second sense electrode spaced apart from the first electrode along a neural pathway of the neural response. A neural response decay is determined, being a measure of the decay in the neural response from the first sense electrode to the second sense electrode. A ratio of the neural response decay to an amplitude normalising term is calculated. From the ratio it is determined whether a locally evoked neural response is present in the neural measurement.

Any discussion of documents, acts, materials, devices, articles or the like which has been included in the present specification is solely for the purpose of providing a context for the present invention. It is not to be taken as an admission that any or all of these matters form part of the prior art base or were common general knowledge in the field relevant to the present invention as it existed before the priority date of each claim of this application.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

In this specification, a statement that an element may be "at least one of" a list of options is to be understood that the element may be any one of the listed options, or may be any combination of two or more of the listed options.

### Summary of the Invention

The present invention provides a system for automated assessment of neural response recordings, the system comprising:
a memory storing a set of basis functions comprising at least one of (a) a compound action potential basis function and (b) an artefact basis function;
an input for receiving a plurality of neural recordings of electrical activity in neural tissue, the neural recordings being obtained by repeated application of stimuli using a single configuration of stimulation and recording; and
a processor configured to decompose each neural recording by determining at least one parameter which estimates at least one of a compound action potential and an artefact from the set of basis functions, the processor further configured to repeatedly determine a plurality of values of the at least one parameter for each respective one of the plurality of neural recordings; and the processor further configured to determine a spread of the plurality of values, and the processor further configured to output an indication that the neural response recordings are of higher quality if the spread is small, and the processor further configured to output an indication that the neural response recordings are of lower quality if the spread is large.

Also disclosed herein, but not claimed as such, is a method for automated assessment of neural response recordings, the method comprising:
storing a set of basis functions comprising at least one compound action potential basis function and at least one artefact basis function;
receiving a plurality of neural recordings of electrical activity in neural tissue, the neural recordings being obtained by repeated application of stimuli using a single configuration of stimulation and recording;
decomposing each neural recording by determining at least one parameter which estimates at least one of a compound action potential and an artefact from the set of basis functions, and repeatedly determining a plurality of values of the at least one parameter for each respective one of the plurality of neural recordings;
determining a spread of the plurality of values; and
outputting an indication that the neural response recordings are of higher quality if the spread is small, and outputting an indication that the neural response recordings are of lower quality if the spread is large.

Also disclosed herein, but not claimed as such, is a non-transitory computer readable medium for automated assessment of neural response recordings, comprising instructions which, when executed by one or more processors, causes performance of the above method.

The indication of the quality of the neural response recordings output by the processor may be a binary indication of either high or low quality, for example wherein the spread is compared to a threshold. Alternatively the indication of the quality of the neural response recordings may be defined on a scale of three or more quality indicia levels, or may be defined on a substantial continuum, from high quality to low quality. For example a quality score may be output and may be normalised to fall anywhere within a desired range, such as [0:1]. Determination of a quality score may be calibrated by reference to clinician scoring of a test set of neural recordings. Similarly, normalisation of the quality score may be calibrated by reference to clinician scoring of a test set of neural recordings, for example the clinician may use the test set to define a midpoint, spread, growth rate or the like of a normalising function such as a sigmoid.

The ECAP quality score may be used to assess a selected configuration of stimulation and recording. A distinct ECAP quality score may additionally be obtained in relation to one or more other configurations of stimulation and recording, for example by altering selection of stimulation electrode(s) and/or selection of recording electrode(s) and generating a new ECAP quality score in relation to the new configuration. Selection of a configuration of stimulation and recording for ongoing therapy may then be made by comparing the quality scores for each configuration. Preferred embodiments may comprise an implant and/or associated clinical software configured to test in an automated manner all possible configurations of stimulation and recording, whereby all implanted electrodes are sequentially used for stimulation, and whereby for each such stimulation configuration all possible recording electrodes are sequentially used to obtain ECAP quality scores for each respective stimulation and recording configuration, so as to produce a matrix or set of ECAP quality scores for the entire implanted electrode array. Such embodiments thus provide an automated means by which an optimal configuration of stimulation and recording may rapidly be identified by referring to the set of ECAP quality scores. Such embodiments may thus save laborious manual clinical efforts, improve the time and cost of optimally fitting a neurostimulator and/or improve therapeutic outcomes for the implantee. Additionally or alternatively, some embodiments may provide for a matrix or set of ECAP quality scores to be produced or updated for some or all possible electrode configurations on an ongoing basis during operation of the implanted device. For example the processor of the implanted device may be configured to produce or update a matrix or set of ECAP quality scores at predefined time intervals, or after a certain number of stimuli have been delivered, and/or at other times as appropriate. On the basis of such ECAP quality scores which are produced during ongoing operation of the device, the device may be configured to adopt an updated stimulation configuration such as a selection of which electrodes to use as stimulation electrodes for ongoing therapy, so as to exploit optimal or preferable ECAP quality scores associated with the updated stimulation configuration. Additionally or alternatively, on the basis of such ECAP quality scores produced during ongoing operation of the device, the device may be configured to adopt an updated recording configuration such as a selection of which electrodes to use as recording electrodes during ongoing therapy, so as to exploit optimal or preferable ECAP quality scores associated with the updated recording configuration.

The spread may be calculated as being the standard deviation of the parameters, a variance of the parameters, an inter-quartile or inter-decile range of the parameters, or may comprise any other suitable statistical measure of data spread.

In some embodiments, the at least one parameter may comprise a correlation of an observed ECAP with a predefined basis function comprising an analytically defined compound action potential basis function, such parameter referred to herein as a Correlation parameter. Such embodiments recognise that in determining the quality of the recording it is advantageous to consider how well the observed ECAP correlates with the analytic or "ideal" ECAP as predefined.

Additionally or alternatively, the at least one parameter may comprise a frequency of an observed ECAP, as measured for example from a time duration of one or more lobes of the observed ECAP and/or from a time offset of ECAP peaks in the recording and/or from spectral analysis of the recording, such parameter referred to herein as a Frequency parameter. Such embodiments recognise that Frequency is a particularly useful parameter to monitor because a large variation in ECAP frequency from one stimulus to the next has been discovered to correlate with poor ECAP signal quality and suboptimal therapy.

Additionally or alternatively, the at least one parameter may comprise a time offset of an observed ECAP relative to a time of the stimulus, such parameter referred to herein as an Offset parameter. Such embodiments recognise that Offset is a particularly useful parameter to monitor because a large variation in ECAP offset from one stimulus to the next has been discovered to correlate with poor ECAP signal quality and suboptimal therapy.

In some embodiments, the basis function is an analytically defined compound action potential basis function. In such embodiments, a rate at which an ECAP is detected in the plurality of recordings may further be used to define a quality of the neural response recordings. Such a rate is referred to herein as a Detection Rate.

In some embodiments, two or more neural recording may be obtained of each ECAP, so that comparative parameters derived from a comparison of the two or more recordings may additionally or alternatively be used to assess ECAP quality. For example, a conduction velocity and/or a dispersion of each ECAP may be determined from two or more neural recordings of that ECAP, and a spread of the conduction velocity and/or a spread of the dispersion may be used to derive ECAP signal quality.

In embodiments where more than one parameter is obtained, the plurality of parameters may be processed by any suitable predefined function to generate a single quality score. For example, in one embodiment, a quality score may be determined as follows:$Score = \left(Detection Rate ∗ Correlation\right) / \left(Frequency spread + Offset spread\right)$

In such embodiments, each element of the function may be scaled or adjusted by any suitable tuning constant or power or the like, to better calibrate outputs to clinicians' opinions. For example when Offset spread is measured in ms, this parameter may be multiplied by 100 in the above function.

Noting that a larger Detection Rate and a larger Correlation correspond to higher ECAP signal quality, preferred functions are proportional to these parameters and/or place these parameters in a numerator of the function. Conversely, noting that a larger spread of Frequency and a larger spread of Offset correspond to lower ECAP signal quality, preferred functions are inversely proportional to these parameters and/or place these parameters in a denominator of the function. Other embodiments may thus utilise any other suitable function aligning with these observations.

In embodiments utilising differential ECAP recording by use of two sense electrodes input to a differential measurement amplifier, some or all of the above-noted parameters may be obtained in relation to both a positive ECAP component of the differential ECAP recording and a negative ECAP component of the differential ECAP recording.

An ECAP signal quality score may be normalised, for example to a range [0:1], by any suitable function, such as a sigmoid function. The Normalised Score may for example be determined by: $Normalised Score = 1 - 1 / \left(1+α*Score\right)$

**In** such embodiments the tuning constant α may be selected so as to calibrate the Normalised Score outputs to clinicians' opinions, and for example in one embodiment α = 800. **In** alternative embodiments α could be replaced by any suitable tuning constant or power or the like. For example, where human clinician assigned scores are selected from "unsatisfactory", "marginal" and "satisfactory", α or other constants may be selected as appropriate in order that the produced Normalised Score is less than 0.4 for at least 90% of signal sets labelled by expert clinicians as 'unsatisfactory'. This presents a threshold independent of implementation that field clinical engineers may refer to when deciding which stimulator configuration to use, whereby a Normalised Score less than 0.4 will indicate that additional programming is required, whilst a Normalised Score greater than 0.6 will predict that the existing stimulation and recording configuration program will produce a clinically usable growth curve. **In** such embodiments, when a Normalised Score between 0.4 and 0.6 is output, the stimulator configuration is considered marginal, meaning that it is unclear whether the stimulator configuration will produce a clinically usable growth curve.

Importantly, embodiments of the present invention recognise that a system for automated assessment of neural response recordings should preferably produce outputs that are insensitive to the stimulation current used. As ECAP amplitude is dependent on stimulation current, this requirement ensures that the system does not incorrectly equate greater ECAP amplitude with greater quality of the stimulation and recording configuration. The parameters chosen in preferred embodiments of the invention advantageously do not depend solely on ECAP amplitude and thus such embodiments do not incorrectly equate ECAP amplitude with quality of the stimulation and recording configuration. It is further to be noted that ECAP magnitude depends on posture, due to both a varying stimulation electrode to nerve distance, and a varying nerve to recording electrode distance, giving another reason why it is advantageous to select parameters which do not solely represent the recorded ECAP amplitude.

References herein to estimation or determination are to be understood as referring to an automated process carried out on data by a processor operating to execute a predefined estimation or determination procedure. The approaches presented herein may be implemented in hardware (e.g., using application specific integrated circuits (ASICs)), or in software (e.g., using instructions tangibly stored on computer-readable media for causing a data processing system to perform the steps described above), or in a combination of hardware and software. The invention can also be embodied as computer-readable code on a computer-readable medium. The computer-readable medium can include any data storage device that can store data which can thereafter be read by a computer system. Examples of the computer readable medium include read-only memory ("ROM"), random-access memory ("RAM"), CD-ROMs, DVDs, magnetic tape, optical data storage device, flash storage devices, or any other suitable storage devices. The computer-readable medium can also be distributed over network coupled computer systems so that the computer readable code is stored and executed in a distributed fashion.

Embodiments of the invention may thus provide a partly or wholly automated process for clinical verification of suitable operation of a neurostimulation device, by reference to ECAP signal quality, using an automated process for testing multiple combinations or all combinations of stimulation variables, in a computationally efficient manner requiring reduced clinical fitting time and expense. In particular, the described embodiments provide processes which exploit data parameters which can be obtained at high speed by a largely automated process, and by exploiting such parameters in particular and avoiding or minimising steps requiring human clinical expert involvement, these embodiments of the invention advantageously avoid the considerable time and expense of a conventional approach involving clinically observing ECAP recordings and/or clinically deriving an ECAP growth curve in each relevant posture in order to identify optimal therapeutic settings for the device. Some embodiments may for example be capable of producing a signal quality score in a fraction of a second, such as within 250 ms and able to be iteratively updated at high speed such as within every 62.5 ms.

Further embodiments of the invention may utilise the signal quality score for ongoing control of operation of a feedback loop of an implanted neuromodulation device. For example, such embodiments may cause a feedback loop to cease operation, or to respond more slowly, at times when an ECAP signal quality score is low. Such embodiments may additionally or alternatively cause a feedback loop to commence operation, or to respond more quickly, at times when an ECAP signal quality score is high.

### Brief Description of the Drawings

An example of the invention will now be described with reference to the accompanying drawings, in which:
Figure 1 schematically illustrates an implanted spinal cord stimulator;
Figure 2 is a block diagram of the implanted neurostimulator;
Figure 3 is a schematic illustrating interaction of the implanted stimulator with a nerve;
Figure 4 illustrates a scrubbing process;
Figure 5 is a signal flow diagram;
Figure 6 illustrates ECAP and artefact basis functions, and their product;
Figure 7 illustrates a system for ECAP and artefact estimation;
Figure 8 illustrates an architecture for a signal quality indicator in accordance with one embodiment of the present invention;
Figure 9 illustrates a clinical system in accordance with an embodiment of the invention;
Figure 10 is a state machine diagram representing an implementation of a measurement electrode scan (MES) in accordance with one embodiment of the invention;
Figure 11 is a flowchart of the MES procedure carried out by the implant;
Fig 12 shows the examples of the MES position configuration methods when stim electrode is E2; and
Figs 13-16 depict example outputs of the MES.

### Description of the Preferred Embodiments

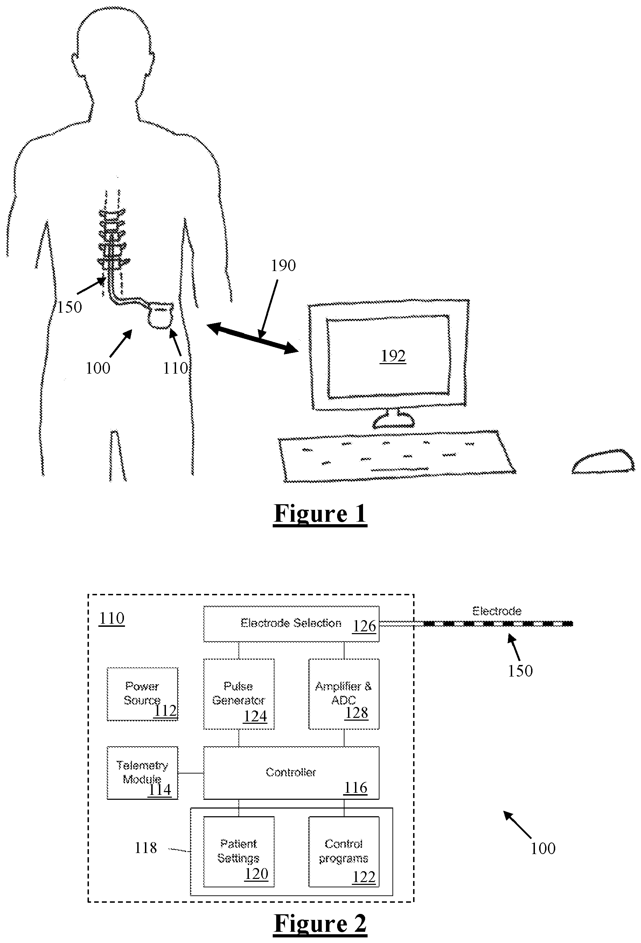

Figure 1 schematically illustrates an implanted spinal cord stimulator 100. Stimulator 100 comprises an electronics module 110 implanted at a suitable location in the patient's lower abdominal area or posterior superior gluteal region, and an electrode assembly 150 implanted within the epidural space and connected to the module 110 by a suitable lead. Numerous aspects of operation of implanted neural device 100 are reconfigurable by an external control device 192. Moreover, implanted neural device 100 serves a data gathering role, with gathered data being communicated to external device 192.

Figure 2 is a block diagram of the implanted neurostimulator 100. Module 110 contains a battery 112 and a telemetry module 114. In embodiments of the present invention, any suitable type of transcutaneous communication 190, such as infrared (IR), electromagnetic, capacitive and inductive transfer, may be used by telemetry module 114 to transfer power and/or data between an external device 192 and the electronics module 110.

Module controller 116 has an associated memory 118 storing patient settings 120, control programs 122 and the like. Memory 118 also stores a set of basis functions comprising at least one of (a) a compound action potential basis function and (b) an artefact basis function, to facilitate fitting or refinement of device operation based on ECAP quality scores. External device 192 also stores a set of basis functions comprising at least one of (a) a compound action potential basis function and (b) an artefact basis function to permit clinical fitting based on ECAP quality scores. Controller 116 controls a pulse generator 124 to generate stimuli in the form of current pulses in accordance with the patient settings 120 and control programs 122. Electrode selection module 126 switches the generated pulses to the appropriate electrode(s) of electrode array 150, for delivery of the current pulse to the tissue surrounding the selected electrode(s). Measurement circuitry 128 is configured to capture measurements of neural responses sensed at sense electrode(s) of the electrode array as selected by electrode selection module 126.

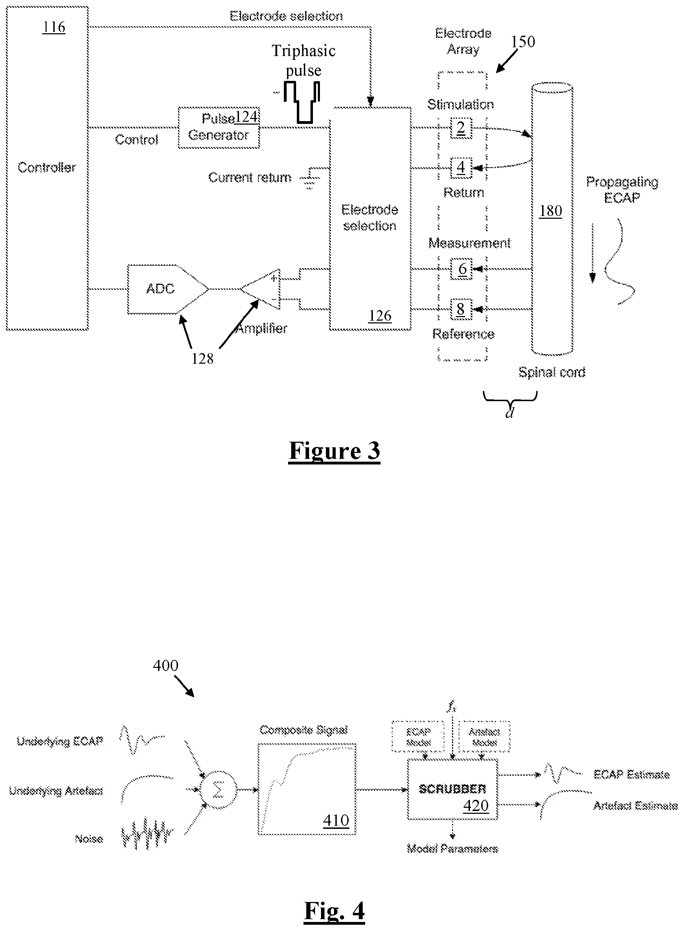

Figure 3 is a schematic illustrating interaction of the implanted stimulator 100 with a nerve 180, in this case the spinal cord however alternative embodiments may be positioned adjacent any desired neural tissue including a peripheral nerve, visceral nerve, parasympathetic nerve or a brain structure. Electrode selection module 126 selects a stimulation electrode 2 of electrode array 150 to deliver a triphasic electrical current pulse to surrounding tissue including nerve 180, although other embodiments may additionally or alternatively deliver a biphasic tripolar stimulus. Electrode selection module 126 also selects a return electrode 4 of the array 150 for stimulus current recovery to maintain a zero net charge transfer.

Delivery of an appropriate stimulus to the nerve 180 evokes a neural response comprising a compound action potential which will propagate along the nerve 180 as illustrated, for therapeutic purposes which in the case of a spinal cord stimulator for chronic pain might be to create paraesthesia at a desired location. To this end the stimulus electrodes are used to deliver stimuli at 30 Hz. To fit the device, a clinician applies stimuli which produce a sensation that is experienced by the user as a paraesthesia. When the paraesthesia is in a location and of a size which is congruent with the area of the user's body affected by pain, the clinician nominates that configuration for ongoing use. This clinical fitting process is conventionally laborious, however the presently described embodiments provide means for automated assessment of the device fitting on the basis of ECAP quality scores, including the stimulation configuration and recording configuration, to improve efficiency of this fitting process.

The device 100 is further configured to sense the existence and electrical profile of compound action potentials (CAPs) propagating along nerve 180, whether such CAPs are evoked by the stimulus from electrodes 2 and 4, or otherwise evoked. To this end, any electrodes of the array 150 may be selected by the electrode selection module 126 to serve as measurement electrode 6 and measurement reference electrode 8. The stimulator case may also be used as a measurement or reference electrode, or a stimulation electrode. Signals sensed by the measurement electrodes 6 and 8 are passed to measurement circuitry 128, which for example may operate in accordance with the teachings of International Patent Application Publication No. WO2012155183 by the present applicant. The present invention recognises that in circumstances such as shown in Figure 3 where the recording electrodes are close to the site of stimulation, stimulus artefact presents a significant obstacle to obtaining accurate recordings of compound action potentials, but that reliable accurate CAP recordings are a key enabler for a range of neuromodulation techniques.

In particular, the recording of ECAPs enables the device to enter a closed loop feedback mode, whereby a target ECAP level is continually sought by the device and whereby the device responds to perturbations in the feedback loop such as postural changes by adjusting future stimulation pulses. However feedback operation depends critically on a quality of the response recordings being obtained by the device. While quality can be reliably assessed by suitably experienced human clinicians, this is laborious. Quality can also be assessed by obtaining a full growth curve for each configuration, representing the growth in ECAP amplitude in response to increasing stimulus current. This allows a check of whether that configuration yields a growth curve with a clear threshold (a stimulus current below which no ECAPs arise), and also whether the growth curve is monotonic increasing above the threshold which is important for feedback loop stability. However, obtaining and assessing a growth curve is also laborious.

The present invention thus provides a system for automated assessment of a quality of neural response recordings.

In more detail, the present embodiment decomposes each neural recording by determining at least one parameter which estimates at least one of a compound action potential and an artefact, using the set of basis functions in memory. This is thus a method for separating composite signals when signal components belong to a closed space of signals that may be represented by distinct basis sets. In neuromodulation this is used to separate the 'ECAP part' and the 'artefact part' of the recorded signals.

A composite signal is a signal that is constructed by the sum of other signals, which will be referred to here as the underlying signals. The basis element signal separation approach of the present invention estimates the underlying signals of the composite signal given only the composite signal, and without knowledge of the exact underlying signals. The present embodiment provides a blind signal separation algorithm which is able to assume some knowledge about the underlying signals. Namely, the present embodiment recognises that it can be assumed that each underlying signal may be represented by a set of basis functions. Unlike blind signal separation algorithms with multiple inputs and one output, the present embodiment produces a deterministic estimate of the underlying signals by leveraging this assumption.

In the field of neurostimulation, a mixed signal may be a combination of an ECAP and stimulus artefact. In some instances, there will be a need to decompose the signals and analyse the components. Analysing the individual components may reveal characteristics of the signal components which may be used in numerous advantageous ways. In some cases, analysing the components of the mixed signals may reveal errors in the system. Further, there may be situations where the mixed or composite signal has a dominant, but superfluous, component masking an essential component. In such cases, the mixed signal must be decomposed into its components, eliminate the superfluous component, and analyse the essential component and the characteristics thereof.

The present embodiment decomposes a mixed signal by determining at least one of the plurality of signals constituting the composite signal from a set of basis functions. The embodiment separates composite signals into their underlying components by modelling each underlying component with a basis. This embodiment may be applied in neuromodulation in the separation of ECAP waveforms form artefact waveforms (as well as noise) given a signal recording which is a mixture of these signals. This yields more robust feature extraction from the ECAP, including the ECAP magnitude which is a feature used by the closed loop control system of Figs 1-3. Additional features such as ECAP peak positions may also be measured more robustly, which is of scientific benefit. The present embodiment estimates both artefact and ECAP simultaneously, where ECAP and artefact signal contributions are balanced to 'best' represent the recorded signal. The present embodiment produces a noiseless ECAP estimate and subject to the definition of the ECAP basis set, can impose certain signal properties (e.g. a baseline of 0V). Further, the present embodiment is efficient (O(n)) and runs in a deterministic time (unlike non-deterministic methods), which means that it may be potentially integrated into firmware, giving improved, real-time ECAP magnitude estimates without the need of a human tuned filter.

**Fig.4** illustrates a scrubber process 400. A scrubber is an algorithm that estimates the ECAP and Artefact components of some composite signal, as depicted at 410. A composite signal is defined as a signal composed of the sum of multiple distinct elements. In the context of ECAP measurement the components of a composite measurement are the artefact, the neurophysiological response to the stimulus (the ECAP), and everything else. The primary goal of scrubber 420 is to isolate the ECAP. However, artefact estimation is usually a by-product of this task and is useful in and of itself as insights into the mechanism of artefact will help us to minimise it in future designs. What is left over consists of electronic noise and neurophysiological noise independent of stimulation.

The present embodiment adopts the following process. Each underlying signal is represented as a linear combination of basis functions. Consider a composite signal with two underlying signals: $σ \left(x\right) = f \left(x\right) + g \left(x\right) \approx {\sum }_{k} {α}_{k} {ϕ}_{k} \left(x\right) + {\sum }_{j} {β}_{j} {φ}_{j} \left(x\right)$

Basis functions are derived empirically based on experience and alternate models of underlying signals. For the purposes of explanation, consider them to be constant. Computing the pairwise inner produces of basis functions and the inner product between each basis function and the composite signal, one may write down a set of linear equations that may be solved with matrix inversion to obtain the sets of coefficients alpha and beta. Given the alpha coefficients, one may then write down the basis representation of *f*(*x*), thus estimating f(x). Similarly, one may estimate g(x) given the beta coefficients. This method is not limited to composite signals containing two components, but the problem it is applied to in the described neuromodulation field has just two components.

The basis element signal separation approach of the present embodiment is a mathematical tool for deconstructing composite signals. Consider a signal containing an ECAP component *f*(*t*) and an Artefact component *g(t).* The signal that we measure in a patient *σ*(*t*) may therefore be expressed as: $σ \left(t\right) = f \left(t\right) + g \left(t\right) + e \left(t\right)$ where *e*(*t*) is some noise. Closed loop stimulation works because the ECAP component of a given signal has a regular shape which resembles two periods of a dampened oscillation. In a similar vein, closed loop stimulation would not work if the artefact component of the signal did not have a regular shape. In order to measure ECAP amplitude we filter out most of the artefact using the detector, which assumes that the artefact has a regular exponential-like shape.

The present embodiment operates on the assumption that ECAP and artefact signal components belong to distinct families of functions. That is, ECAPs are always short oscillatory events, whilst artefacts are exponential-looking signals. For each distinct family of functions we can predefine a basis to represent it. For suitable basis functions, the basis coefficients can be calculated and the ECAP and artefact basis expansions can each be isolated. The ECAP basis expansion then provides us an estimate of the ECAP component, free from artefact.

The calculation of basis coefficients balances the contributions of each of the basis functions in such a way that the overall signal is approximated as best as possible. In other words, the estimated ECAP and Artefact contributions are balanced so as to best model the signal that has been recorded. In order to do achieve better performance, the present embodiment assumes that all ECAPs belong to a certain family of functions and that ECAP shapes outside of this family do not exist. At the time of writing, ECAPs with late responses such as those set forth in WO2015070281 are outside the family of ECAP functions used by the present embodiment and therefore cannot be estimated properly. Therefore, other Scrubbers may be more appropriate to use when working with signals not adequately modelled by the ECAP basis in use at the time.

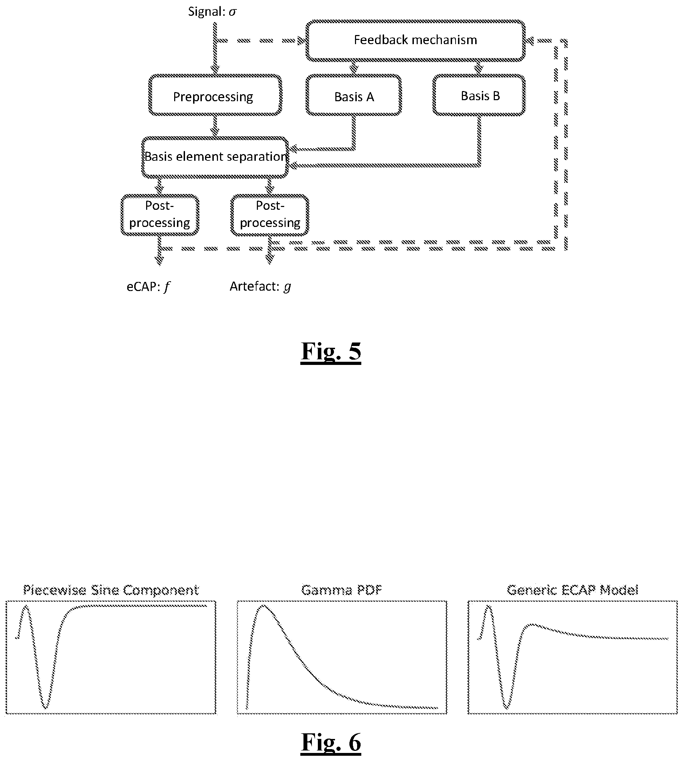

The method described above forms the block in the signal flow diagram of **Fig. 5****.** Pre and post processing are used, in some embodiments, to improve signal estimates. For example, pre-processing can be used to reduce high frequency noise in the signal. The feedback mechanism however is used to improve the construction of basis sets. A crude 'first guess' basis may be used to approximate the signal and the estimates that are produced can be used to refine the basis set on subsequent passes. For example, the first pass might guess an ECAP basis in order to get a good estimate of the artefact. Subtracting the artefact from the signal and using signal correlation methods can be used to refine the choice of ECAP basis. Re-running the algorithm with the improved basis will yield better estimates of both the ECAP and the artefact.

Artefact is modelled by the present embodiment using three basis functions: ${ϕ}_{1} \left(t\right) = 1 , {ϕ}_{2} \left(t\right) = t , {ϕ}_{3} \left(t\right) = exp \left(-\frac{16.384 \times {10}^{3}}{7.0}t\right)$

The unit basis function *ϕ₁* captures the DC content of the measured signal. The linear basis function *ϕ₂* captures the component of Artefact due to amplifier drift. The exponential basis function *ϕ₃* captures the chemical charge relaxation component of the Artefact. The decay constant of the exponential component can be any suitable variable and the value above was determined empirically based on model performance against a library of human Artefact recordings. Different devices may present different artefact and/or ECAP outcomes and may consequently require different constants, which can be similarly empirically obtained.

Once the algorithm of the present embodiment is applied, the Artefact component of the signal is represented by: $A \left(t\right) = {αϕ}_{1} \left(t\right) + {βϕ}_{2} \left(t\right) + {γϕ}_{3} \left(t\right)$

This model, while simple, has been applied to many thousands of representative human patient neural recordings and has been found to perform well. In combination with the ECAP basis functions, the combined model accurately estimates the recorded signal.

Unusual neurological Artefact such as background neuronal activity or late response are not modelled in the present embodiment, but may be incorporated in accordance with alternative embodiments of the invention. Estimates obtained from the approach of the present embodiment will remove such features and therefore the outcome cannot be relied upon in the measurement of non-ECAP neurological features, at least in this embodiment.

An ECAP basis function is defined using the product of a Gamma probability density function, with parameters k = 1.7 and θ = 0.60, $φ \left(t\right) = {\left(ft\right)}^{k - 1} ⋅ {e}^{- ft / θ}$

This is a piecewise function composed of one period of a sine wave followed by an exponential function such that the derivative is continuous at their boundary: $ϕ \left(t\right) = \left\{\begin{matrix}0 & t < arcsin \left(C\right) / 2 πf \\ sin \left(2πft\right) - C & arcsin \left(C\right) / 2 πf < t < 1 / f \\ 1 - C - {e}^{- 2 πf \left(t-1/f\right)} & t > 1 / f\end{matrix}\right.$ where C = 0.37. The two components and their product are represented in **Figure 6****.** There is only one morphology parameter in this FPAP model; the frequency of the sinusoidal component: f . As can be seen above, the timescale of the Gamma PDF is scaled accordingly. This model was arrived at through the hand fitting of elementary functions to simulated ECAP models.

By scaling the time axis by v and applying an offset t₀: *v* (*t - t₀*)*,* we can stretch and shift an ECAP basis function in time. Let such a stretched and scaled ECAP be called a parametric ECAP basis function: *φ_{v,t0}*(*t*)*.*

There are two distinct ECAP models. One for singled ended measurements and another for differential measurements. The single ended ECAP basis consists of one parametric ECAP basis function and the ECAP E is represented by: $E \left(t\right) = {κφ}_{ν , {t}_{0}} \left(t\right)$

The differential ECAP basis is formed by the difference of two parametric ECAP basis functions giving the following ECAP model $E \left(t\right) = {κ}_{+} {φ}_{{ν}_{+} , {t}_{0 +}} \left(t\right) - {κ}_{-} {φ}_{{ν}_{-} , {t}_{0 -}} \left(t\right)$

In either model, the time stretch (corresponding to the ECAP oscillation frequency) and the time offset are chosen such that κ or κ+ is positive and x- is negative. A sweep of ECAP frequencies and offsets are tested by the present embodiment to ensure this condition holds. The frequency and offset selected to model the ECAP component of a recorded signal are chosen such that the fit to the recording using both ECAP and Artefact models is as good as possible.

It should be noted that the single ended ECAP model assumes fixed ratios between peak heights and peak times. Neurophysiological parameters such as width at half height or the *n₁ : p₂* ratio are entirely determined by the temporal stretch *v* applied to the parametric basis function.

As with Artefact, this assumption has been validated by fitting parametric basis functions to real-world single ended measurements.

In the case of the differential model, such neurophysiological parameters are able to vary independently of *v*+ and *v*- and additionally depend on the composition of the ECAP estimate. That is, κ+ and κ- provide additional degrees of freedom. Although relative neurophysiological parameters are able to vary they have restricted freedom compared to more free-form ECAP models. As with the single ended ECAP assumption, this model constraint has been validated by fitting the differential ECAP basis to real-world differential measurements.

The range of parametric ECAP frequencies is limited to a linearly spaced set of frequencies between 500Hz and 2kHz. The upper limit of 2kHz was chosen to minimise the interference of broad spectrum (up to 8kHz) noise on the parameter selection procedure. The lower limit of 500Hz was chosen to limit the interference of the Artefact on the parameter selection procedure. A slow enough parametric ECAP will closely resemble Artefact in a confined window of time. The range of offsets that are tested was chosen to be significantly wide to model real-world ECAPs, but reasonably constrained to maintain computational performance.

Up until this point, we have assumed that each recorded signal contains an ECAP. However, in practice this is never the case for signals that are sub-threshold, that is, where the applied stimulus was insufficient to recruit any neural response, so that the recorded signal necessarily does not include any ECAP in such circumstances. Including ECAP basis functions in the model for a sub-threshold signal poses a problem, as an ECAP would be fitted to the noise in the signal and the estimate would be meaningless. Additionally, the Artefact component of the signal would be misrepresented as ECAP and Artefact features are balanced in a combined model.

It is therefore desirable to include a mechanism that detects the presence of ECAP in a signal so that the ECAP basis may only be included in the overall model when an underlying ECAP is authentic. The present embodiment incorporates such a mechanism. The signal is modelled using an Artefact only basis and a combined ECAP and Artefact basis. A set of signal features is derived from the estimates produced by both models and combined with signal features from the recorded signal. A series of signals known to contain both ECAP and Artefact or just Artefact were analysed by the present embodiment and the derived set of features saved. Machine learning is used to train a classifier with categories: 'ECAP' or 'no ECAP' . After sufficient training the resulting classifier is able to automatically judge the presence of ECAP in a signal. The present embodiment is rated to detect ECAP in signals containing ECAP with an accuracy of 85% and to reject ECAP in signals containing only Artefact with an accuracy of 95%.

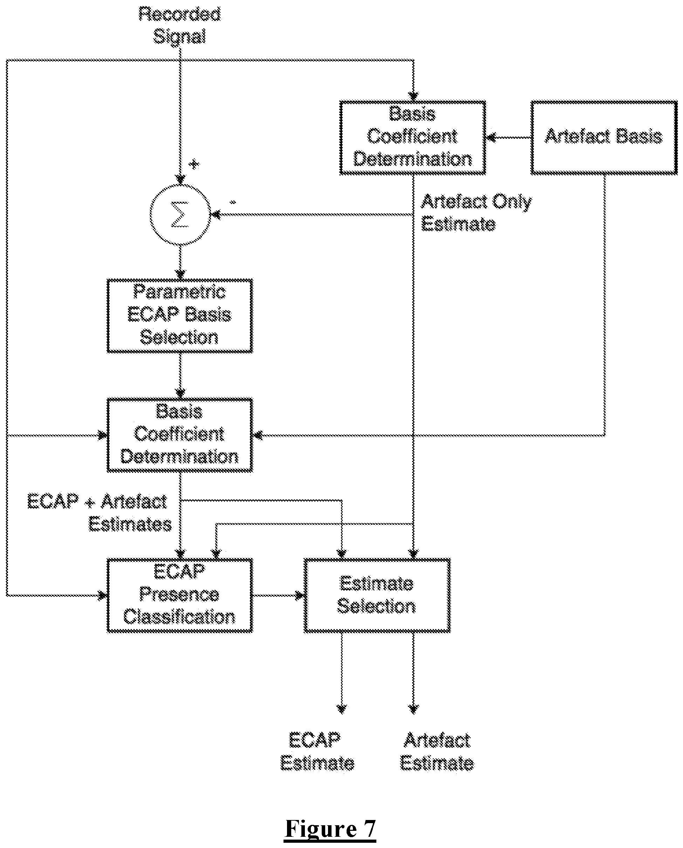

Combining these concepts together, we arrive at the complete algorithm of the present embodiment as depicted in **Fig. 7****.**

The recorded signal is first modelled using an Artefact only basis, under the assumption that it contains no ECAP. Regardless of ECAP presence this will provide an estimate of the Artefact via the basis coefficients. If an ECAP is present this estimate may be refined by including an ECAP basis as well. The initial Artefact estimate is subtracted off the recorded signal to help better determine the parametric ECAP basis. The estimated Artefact and derived features are passed to the 'ECAP Presence Classification' (or ECAP detector) block for later use.

Once the parameters for the Parametric ECAP Basis are determined, the coefficients of the ECAP and Artefact basis in conjunction are then determined. Resulting estimates and feature sets passed to the ECAP detector.

The ECAP detector now has everything it needs in order to classify the presence of ECAP in the recorded signal. Based upon its decision, either the ECAP and Artefact estimates are returned or the Artefact only estimate is returned.

The method steps are as below:
a.Capturing/ recording a composite signal, wherein the composite signal has two or more additive components
b. Selecting a first basis set, corresponding to the first signal component, from a pool of basis sets. Selecting a second basis set, corresponding to the second signal component, from a distinct pool of basis sets.
c.Determining a first component and the second component of the composite function based on the bases functions. Determining an estimate for the first component as a linear expansion of the first basis set, and an estimate for the second component as a linear expansion of the second basis set.
d. Iteratively improving the basis sets using the estimated components from the previous iteration.

The following explanations delve into the mathematics behind the present embodiment. Coefficient Determination is as follows. Let *σ*(*t*) be the signal we record, and *f(t)* and *g(t)* the underlying ECAP and Artefact components respectively. The problem we are attempting to solve is to find estimates for *f*(*t*) and *g(t),* which we do not know, using the recorded signal *σ*(*t*)*,* which we do know. For simplicity, we assume there is no noise in the signal. Therefore, $σ \left(t\right) = f \left(t\right) + g \left(t\right)$

Now suppose that *f*(*t*) may be represented using a finite set of basis functions {*φₖ*(*t*): *k* ∈ {1, 2, ... *n*}}. Similarly, suppose that *g(t)* may be represented using a finite set of basis functions {*ϕⱼ*(*t*)*: j* ∈ {1, 2, ... *m*}} all distinct from the set used to represent *f*(*t*). Then *f*(*t*) and *g(t)* may be expanded over their respective bases, $f \left(t\right) = {\sum }_{k = 1}^{n} {a}_{k} {φ}_{k} \left(t\right)$ $g \left(t\right) = {\sum }_{j = 1}^{m} {b}_{j} {ϕ}_{j} \left(t\right)$

Then by simple substitution: $σ \left(t\right) = {\sum }_{k = 1}^{n} {a}_{k} {φ}_{k} \left(t\right) + {\sum }_{j = 1}^{m} {b}_{j} {ϕ}_{j} \left(t\right)$

At this stage of the problem, the basis sets are known but the coefficients for the specific signal *σ*(*t*) are not. With the coefficients we may recover estimates for *f*(*t*) and *g(t).* We will recover them now.

Consider the following functional inner product for any basis function of *f*: *φᵢ*(*t*) and by the linearity of inner products we have: $\left〈σ\left(t\right),{φ}_{i}\left(t\right)\right〉 = {\sum }_{k = 1}^{n} {a}_{k} \left〈{φ}_{k}\left(t\right),{φ}_{i}\left(t\right)\right〉 + {\sum }_{j = 1}^{m} {b}_{j} \left〈{ϕ}_{j}\left(t\right),{φ}_{i}\left(t\right)\right〉$

Similarly, consider the functional inner product for any basis function of *g*: *ϕₗ*(*t*) $\left〈σ\left(t\right),{ϕ}_{l}\left(t\right)\right〉 = {\sum }_{k = 1}^{n} {a}_{k} \left〈{φ}_{k}\left(t\right),{ϕ}_{l}\left(t\right)\right〉 + {\sum }_{j = 1}^{m} {b}_{j} \left〈{ϕ}_{j}\left(t\right),{ϕ}_{l}\left(t\right)\right〉$

Equations (4) and (5) provide us with a system of *n + m* linear equations with *n + m* unknowns (the coefficients *aₖ* and *bⱼ* ). Thus, determining the coefficients is a matter of solving a linear equation: $Hν = b$ where $H = \left(\begin{matrix}\left〈{φ}_{1}, {φ}_{1}\right〉 & \left〈{φ}_{1}, {φ}_{2}\right〉 & ⋯ \\ \left〈{φ}_{2}, {φ}_{1}\right〉 & ⋱ & ⋮ \\ ⋯ & ⋯ & \left〈{ϕ}_{m}, {ϕ}_{m}\right〉\end{matrix}\right) , b = \left(\begin{matrix}\left〈σ, {φ}_{1}\right〉 \\ ⋮ \\ \left〈σ, {ϕ}_{m}\right〉\end{matrix}\right)$

Thus the coefficients may be solved via *H*⁻¹*b.* The matrix *H* is invertible if and only if none of the basis functions from the ECAP basis belong to the span of the Artefact basis and vice versa, and basis functions with ECAP and Artefact bases are distinct. Basis functions should be scaled to unit power so that comparatively large or small inner products do not introduce computational error during the inversion of *H.*

In practice there is noise in the signal which is not modelled by either basis. However, introduced errors will be minor since the inner product of an independent noise source and any signal is zero for an inner product taken over an infinite time interval. Limiting the inner product to a finite number of samples when calculating *b* will propagate some error, however, this error is not significant.

ECAP Parameter Determination is as follows. The parametric ECAP basis is determined using the recorded signal with the initial Artefact removed and any residual baseline subtracted. Let this signal be called the 'refined recording'. A correlation mesh is determined by sweeping a range of basis ECAP frequencies and offsets and taking the dot product between the refined recording and each parametric basis function.

For single ended and differential mode, the present embodiment samples 16 linearly spaced frequencies between 800Hz and 2kHz and offsets from -7 samples to -1 samples inclusive. This range of frequencies and offsets was found to work well against test signals observed in human subjects but these ranges may be extended. Extending them too far will allow the parametric ECAP to lock onto noise or the Artefact so do so with caution. The highest positive stationary point of the correlation mesh determines the parameters of the first ECAP basis element. If the measurement is single ended, then this is the only ECAP basis element.

In the case of a differential ECAP measurement, a new correlation mesh is calculated, sampling 16 linearly spaced frequencies between 500Hz and the frequency of the previously determined basis element. It is assumed that the reference is always further away from the stimulus than the recording electrode. This allows us to exploit human neurophysiology since ECAP frequency monotonically decreases with recording distance. In a similar vein, offsets are tested between the previous ECAP basis offset and 12 samples. Again these ranges were empirically chosen to work well with good signals from humans. Instead of using the highest positive stationary point of the correlation mesh, the most negative stationary point instead determines the parameters of the secondary basis function. If there are no negative stationary points, only the primary basis function is utilised.

The majority of blind signal separation algorithms assume that the underlying signals are statistically independent and use statistical signal processing techniques to estimate the underlying signals. The problem of ECAP and artefact estimation cannot be solved in this way because the underlying signals are fundamentally dependent on one another. Instead the present embodiment assumes that each underlying signal may be expressed as a linear combination of basis functions (a stronger assumption) limiting its application to processes where there is already some knowledge of the underlying signals before they are recorded in the form of a composite signal.

The Artefact Model lists the basis functions used to model the Artefact present in our hardware/recordings. The FPAP model is a singular basis function used in the total ECAP basis set. In practice we use one FPAP for single ended measurements and two FPAPs for differential measurements to take care of the reference electrode effect arising with differential measurements taken between two recording electrodes.

Alternative embodiments are further provided. In this embodiment the process of Fig. 4 is instead implemented as follows.

An Artefact Estimation Scrubber is a Scrubber that attempts to estimate only the Artefact component of the signal g(t) and derives an ECAP estimate using σ(t) - g(t). Exponential Scrubbers model the Artefact as the sum of exponential functions. There are three such models envisaged here:

**Table 1: The Exponential Scrubber Artefact models**

| | Exponential Time domain representation |
|---|---|
| Single | *g*(*t*) = *a* exp(*-bt*) + *h* |
| Double | *g*(*t*) *= a* exp(-*bt*) *+ c* exp(*-dt*) + *h* |
| Triple | *g*(*t*) = *a* exp(-*bt*) + *c* exp(-*dt*) x + *f* exp(-*gt*) + *h* |

A non-linear optimisation is performed using the simplex hill-climbing Nelder Mead algorithm where the parameters a; b; c; d; e; f; g and h are all tuned to minimise the value of a cost function. The non-linear optimisation minimises the sum of the squares error between the estimated Artefact samples and the samples of the recorded signal. Mathematically, the cost function is defined as: $E \left(g, σ\right) = {\sum }_{i = 0}^{n} {\left(σ\left[i\right]-g\left[i\right]\right)}^{2}$

Non-linear optimisations are non-deterministic algorithms, meaning that they do not terminate in a predictable or pre-determinable amount of time. That means that it is possible to provide such a scrubber with a signal that cannot be scrubbed in a reasonable time frame. Further, non-linear optimisations can become stuck in local minima, failing to find the true optimal solution. In practice, this Scrubber works well but it has limitations that should be known before putting it to general use. Nevertheless such embodiments do have uses in certain applications.

A further embodiment is a fractional pole Scrubber works on the same principles as the exponential Scrubbers where a non-linear optimisation is used to determine parameters a; k; α and h of the following Artefact model: $g \left(t\right) = a exp \left(-kt\right) ⋅ {t}^{1.0 - α} + h$

Yet another embodiment is a Complex Pole Scrubber. If we assume that the artefact is a second order response (a double exponential is a subset of this kind of response), then we can estimate the parameters of the second order response that fits the raw signal. For discrete signals, the artefact g follows the model: $g \left[n\right] = b ⋅ g \left[n-1\right] + c ⋅ g \left[n-2\right]$

Given a sequence of samples we may write down the matrix equation: ${g}^{⇀} = A \left(\begin{matrix}b \\ c\end{matrix}\right)$ where, $\vec{g} = \left(\begin{matrix}g \left[n\right] \\ g \left[n-1\right] \\ ⋮\end{matrix}\right) , A = \left(\begin{matrix}g \left[n-1\right] & g \left[n-2\right] \\ g \left[n-2\right] & g \left[n-3\right] \\ ⋮ & ⋮\end{matrix}\right)$

The coefficients b and c may therefore be determined by computing: $\left(\begin{matrix}b \\ c\end{matrix}\right) = {\left({A}^{T} A\right)}^{- 1} {A}^{T} \vec{g}$

The preceding analysis then feeds into an algorithm called a Signal Quality Indicator (SQI) that assigns a quality score to a set of ECAPs recorded under the same stimulator program. Such algorithm may be used in signal quality indicators in clinical data analysis software and clinical user interface software.

In order to build a system for automated assessment of the quality of a signal, the properties of a signal that make it 'good' as opposed to 'bad' must be defined. Test cases on the spectrum of 'good' to 'bad' may then be used to assess the performance of an SQI. However, no such definitions of signal quality exist because it is unclear what properties of individual signals lead to poor clinical results in closed loop spinal cord stimulation. In contrast it is relatively easy to assess the quality of a growth curve, which is a known indicator of clinical success for a closed loop patient.

Therefore, the quality of a group of signals recorded under the same stimulator configuration is defined as the prediction of the quality of the growth curve that would be measured using the same stimulator configuration. However, growth curves are time consuming to collect. Objective guidance prior to growth curve collection on which programs will yield satisfactory growth curves is therefore sought after by field clinical engineers.

A stimulator program is defined as the combination of the stimulation waveform parameters, stimulation frequency and electrode arrangement. Signals are measured with the same stimulator program when these quantities are kept constant. The stimulation current may vary across signals because the present embodiment operates under the assumption that ECAP morphology does not change with stimulation current, and that only the peak to peak magnitude of the ECAP varies with current.

The Signal Quality Indicator (SQI) of the present embodiment assesses the quality of multiple signals recorded with the same stimulator program in open loop mode (i.e. feedback not enabled), and outputs a measure of predicted growth curve quality as a single number between 0 and 1. A higher score indicates that signals recorded with said program are of a higher quality and are more suitable for use in growth curve measurement. Multiple recordings (or signals) are required to perform an assessment because quality estimates should be robust to individual signals of unusual quality. Instead it is desirable for the SQI to provide an indication of the general signal quality of a stimulator program.

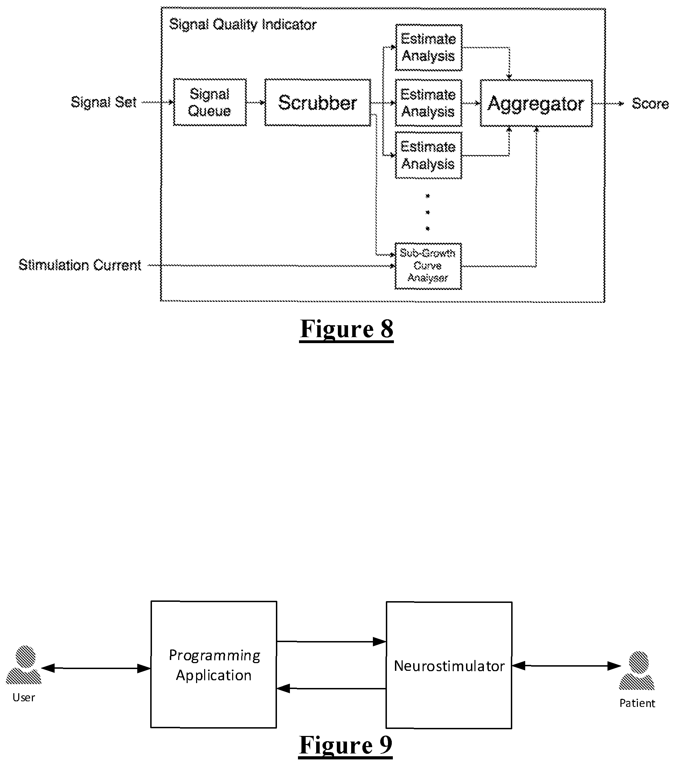

**Figure 8** depicts the architecture of an SQI system in accordance with one embodiment of the invention.

It is to be noted that alternative embodiments may derive an ECAP signal quality score by reference to reference ECAPs which are derived by other means. For example, a residual signal may be obtained by subtraction of an artefact estimate from a recorded signal, and may simply be compared to a clinically verified template ECAP saved in the device since a time of fitting. The clinically verified template ECAP may for example comprise an ECAP recording obtained significantly above threshold to improve SNR, and verified by a clinician as being suitable to be stored in the device to serve as such a template.

Growth curves of varying quality were scored for their usability in closed loop SCS therapy by experienced clinicians. Subsets of signals recorded with same program used to produce these growth curves were used as a Signal Quality Test Library. Performance of an SQI is assessed by its ability to produce quality scores that give consistent rankings with those assigned to the programs in the Signal Quality Test Library. Algorithm tuning/learning was not performed on the Signal Quality Test Library, but rather on a Signal Quality Training Library.

The SQI receives each input signal as a list of samples. The SQI also receives the stimulation current alongside each input signal. The SQI produces a quality estimate upon receiving 4 or more signals as input, using multiple signals. The SQI is used to assess the quality of a program so that high quality programs may be more easily selected for clinical use.

Additionally, quality might be assessed by measuring the consistency of the estimated ECAP component of a signal. Inconsistent estimates indicate that either signal quality is poor and consequently ECAP estimation is poor or that the modelling of the signal components is poor, as may occur when presented with degenerate signals. The SQI outputs a quality estimate in the form of a decimal number between 0 and 1.

The intention of a quality indicator is to enable FCEs to find good programs for patients faster without having to rely upon experience and developed intuition about signal quality. Presenting multiple outputs may reduce the mental/experiential burden placed on FCEs but will still require training or developed intuition in aggregating the meaning of multiple indicators. Providing a single indicator, as is provided by the present embodiment of the invention, is therefore desired.

The approach of the present embodiment can be represented in pseudo code by: $\begin{matrix}score = \left(scoreParams.DetectionRate*scoreParams.MeanPositiveCorrelation\right) \\ / \left(\begin{matrix}\left(scoreParams.StdPosFreq+100*scoreParams.StdPosOff\right) + \\ 30 / \left(scoreParams.DetectionRate+1e-3\right)\end{matrix}\right) ;\end{matrix}$ $\begin{matrix}return 1.0 - 1.0 / \left(1.0+alpha*score\right) ; / / converts a score from \left[0, inf\right] into a score \\ from \left[0, 1\right]\end{matrix}$

The signal quality indicator (SQI) is a tool used to guide FCEs in the selection of programming parameters. The SQI is a number between 0 and 1 which, in conjunction with SQIs measured across different patient programs, provides insight into which of those programs will perform the best. For example, if Program A has an SQI of 0.9 and Program B has an SQI of 0.5, the clinical engineer would opt for Program A. In this sense the SQI can be considered to be a predictor of patient outcome.

Signal quality may be interpreted in one of two ways: objective and subjective. Objective signal quality is represented by objective signal properties such as signal to noise, which no amount of signal processing can remove. Subjective signal quality is a measure of how much information can be extracted from a signal given the capability of the implant in use. This subjective signal quality category covers signal features such as signal to artefact ratio. Ideal artefact removal approaches not limited by processing time and capacity can improve subjective signal quality, but given the limited filter capability available in a practical implant and in practical clinical programming sessions, the present embodiment instead makes a prediction of patient outcome within the constraints of such applications. The signal quality indicator used in various embodiments of the invention can involve a combination of objective and subjective signal qualities. Under the assumption that the neurophysiological response varies only in amplitude across time but not in morphology, a subjective SQI will take into account the variability of certain signal features, thus requiring a time sequence of signals. An objective SQI however, may produce a score based on individual signals.

The SQI of the described embodiment is derived from a time sequence of signal features. The features utilised are:
- ECAP detection, as determined by the basis element signal separation mechanism described in the preceding;
- Model parameters, also as estimated by the mechanism described in the preceding fitting methods;
- model correlation, also as computed by the mechanism described in the preceding; and
- stimulus current.

Given the time sequence of features, the derived SQI time sequence is determined. The present embodiment provides for signal quality indicators that vary over different time scales. Estimates of the variability of certain signal features require some sample size before an estimate may be produced. Using a small sample size will provide a fast updating SQI compared to a large sample size. The fast updating SQI used by the present embodiment is defined as follows: $s = \frac{\overline{r} ⋅ {\overline{x}}_{+}}{α Var \left({f}_{+}\right) β {Vard}_{+}}$ where *-r̅* ∈ [0, 1] is the rate of detection, *x̅* + ∈ [0, 1] is the average correlation measured between the scrubbed signal and the selected reference electrode ECAP model, *f*+ and *d+* are, respectively, the frequency and delay parameters estimated for the reference electrode ECAP model, and α and β are empirical constants used to appropriately weigh the contributions of the variance estimates.

Signal statistics are computed over 32 samples, requiring at least 32 signals before the first SQI score s may be computed. After this step the score s is not confined to the specified range of [0; 1] but instead can extend out to ∞ if both parameter variances are 0. Accordingly, in a next step a normalisation is applied to s, to produce a Normalised Score, as follows: $s ′ = \frac{1}{1 + γexp - τs}$

Now s' ∈ [0, 1]. The constants γ and τ have been tuned using clinical experience to give the greatest differentiation between quality scores in a clinical setting. If these parameters are incorrectly chosen, scores will inappropriately tend to reside close to 1 or close to 0 for a majority of the time.

A slow updating SQI is also utilised. The benefit of a slowly changing SQI is that scores are assigned over a long history of signals and are not overly sensitive to local signal changes. As such, the clinical engineer will have scores that are stable and will be better equipped to choose a program as compared to SQIs that constantly change the 'best' choice of program based on local signal properties. A slow varying SQI may be obtained by increasing the sample size above. However, in this embodiment, a weighted ensemble average is adopted. Every n(= 32) samples, s' is computed. A slow varying SQI is then derived from the weighted average: $s " = \frac{{\sum }_{0}^{M} {i}_{n}^{-} \left(nk\right) ⋅ s ′ \left(nk\right)}{{\sum }_{0}^{M} {i}_{n}^{-} \left(nk\right)}$
where *iₙ*(*j*) is the average current at timepoint *j* taken over the past *n* samples, s" represents the historical evolution of s' but weighted by current. The motivation for weighting quality by current is that objective signal quality is expected to improve as current is increased as the size of the neurophysiological response with respect to the noise floor is expected to increase. Alternative embodiments could use any other program parameter to define a weighted average in such a way based on the knowledge that said program parameter is known to improve the objective or subjective signal qualities.

In one embodiment the system is configured so that in the clinical setting, signal quality is presented for four different patient program alternatives and each quality score is configured to evolve as new signals are observed. The number display for each quality score is scaled to a percentage between 0 and 100 and the clinical engineer may use the SQI prediction to narrow in on a patient program prior to enacting a closed loop control programming procedure and assessing clinical efficacy.

Alternative embodiments of the invention could similarly implement an SQI derived from any time sequence of signal features including Signal to Artefact Ratio (SAR), Signal to Noise Ratio (SNR) or frequency domain features such as spectral peak positions. The time sequence of other device program parameters may also be included in the signal quality estimate in some embodiments.

Embodiments of the present invention may thus be of particular assistance in automating programming of the device for each individual patient as much as possible.

Embodiments of the invention may provide particular benefits in relation to neuromodulation utilising closed loop feedback on the basis of observed outcomes, such as ECAP amplitude. In such feedback systems, a possible behaviour of the loop is that if the ECAP signal is lost or the signal to noise ratio becomes too low in some way (e.g. due to significant lead migration or an additional noise source) and the measured ECAP amplitude is reduced due to such effects (but not necessarily due to an actual reduction in recruitment), then the system will increase the stimulus current in order to bring the measured ECAP amplitude back up to a specific target. This can result in excess recruitment. Moreover, in the event of total loss of ECAP measures, the feedback loop will operate to increase the stimulus current until it either hits the Maximum Current Limit, or the compliance voltage limit. Either of these endpoints can result in some discomfort to the patient, and more dorsal column activation than intended. In the opposite case, if the ECAP amplitude measured is higher than actual recruitment for some reason, the current will be driven to 0 mA and the patient will not get any therapy and/or may feel intermittent stimulation, which is often frustrating and uncomfortable. By integrating ECAP signal quality determination in accordance with the present invention into such a feedback loop, the feedback loop operation can be improved by modifying the loop in a manner to restrain or preventing such undesirable loop excursions from occurring if the ECAP signal quality is low. For example, a simple step would be to halt feedback loop operation entirely at times when the ECAP signal quality is below a threshold, and to resume feedback loop operation at times when the ECAP signal quality is above that threshold or another threshold. The patient may be notified of such occurrences.

**Figure 9** illustrates a clinical system in accordance with one embodiment of the invention, in which the programming application associated with a clinician user arranges for the neurostimulator to carry out an automated scan of all possible configurations of the recording electrodes, to thereby obtain a matrix or set of ECAP quality scores for all possible electrode configurations.

The automated scan is also referred to herein as a measurement electrode scan (MES). Notably, the MES is executed by the implanted device in this embodiment, which allows for more rapid execution of the automated MES, thereby hastening clinical fitting and also minimising the chance that patient postural changes may affect the comparative results.

The results of the MES are presented visually by the programming application so as to allow the clinician user to see in real time a signal quality indication (SQI) for multiple electrode locations. In particular, the programming application is configured to also visually present the estimated neural response to the stimulation as measured in a currently selected stimulation and recording configuration, but also simultaneously presents a SQI for multiple alternatives which the clinician may wish to consider.

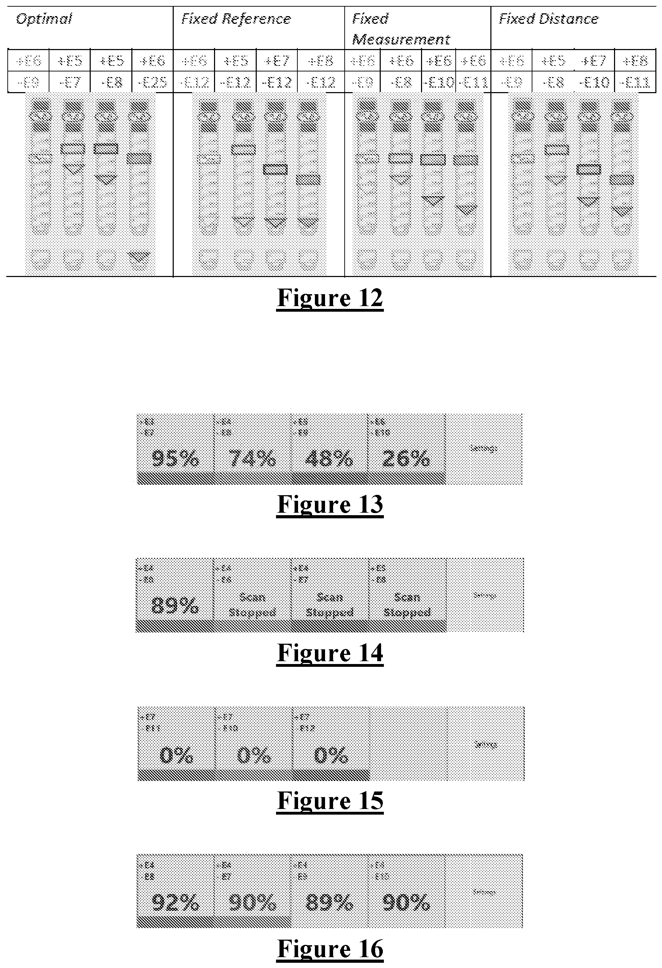

The measurement electrode scan allows ECAPs from multiple electrode configurations to be displayed at the same time. It is intended to assist in optimizing the choice of measurement electrodes and settings. By default, the measurement electrode scan will be automatically started when stimulation is started. The measurement electrode scan consists of up to four measurement electrode configurations. the configuration selected by the user and three other configurations. The electrodes used in the scan are based on the location of the stimulation, measurement and the reference electrodes that are selected in the electrode display window, refer to Fig 12.

The settings to choose electrodes and perform MES are as below:

| **Button** | **Action** | | |
|---|---|---|---|
| *Clear SQI* | Clear the SQI values and restart the SQI calculations. The SQI will be displayed as '-' until ECAPs are received. | | |
| *Stop* / *Start Scan* | Stop or Start the measurement electrode scan while stimulation is running. This does not change the automatic start of the scan when stimulation is started. | | |
| *Position Configuration* | Choose the method used to select up to 3 electrode configurations to be used in the scan These configurations are in addition to the user selected configuration (for examples see Figure 7.5). | | |

| | **Button** | **Measurement** | **Reference** |
|---|---|---|---|
| | *Optimal** (#electrodes from stimulation) | 3 | 5 |
| | | 3 | 6 |
| | | 4 | 7⁺ |
| | | 4 | case (E25) |
| | *Fixed Measurement^* {#electrodes from selected) | As selected | 1 closer |
| | | | 1 further |
| | | | 2 further |
| | *Fixed Reference*^ {#electrodes from selected) | 1 closer ‡ | As selected |
| | | 1 further | |
| | | 2 further | |
| | *Fixed Distance^* (#electrodes from selected) | 1 closer ‡ | 1 closer |
| | | 1 further | 1 further |
| | | 2 further | 2 further |
| *Disable* / *Enable Scan* | Disable or Enable the measurement electrode scan for the duration of the programming session. When disabled, the measurement electrode scan will not be automatically started when stimulation is started. This control is also available in the CLS menu (see Section 8.4). | | |

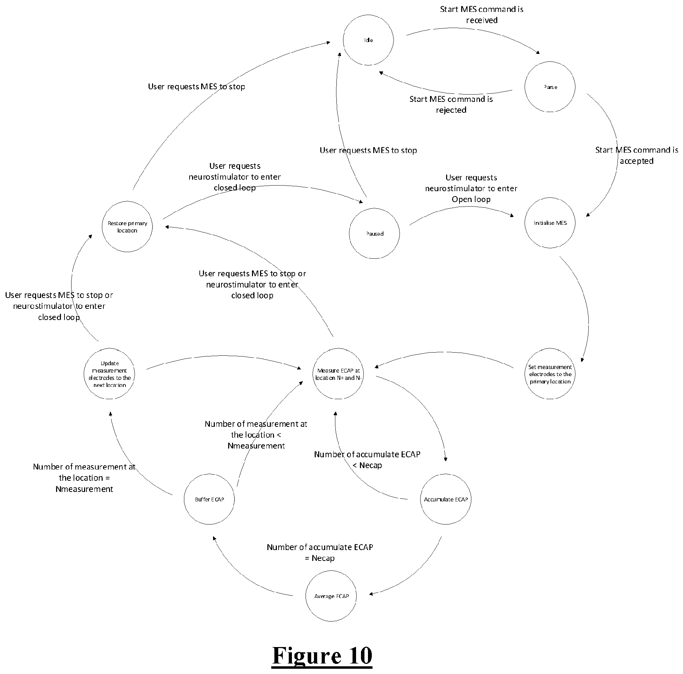

**Figure 10** is a state machine diagram representing an implementation of the MES in accordance with one embodiment of the invention. The primary location is defined as the location used by the neurostimulator to calculate the neural response to stimulation. Necap is defined as the number of measurement to be used for the averaging of the ECAP. In the case of averaging being disabled, Necap is equal to 1. Nmeasurement is defined as the number of averaged ECAP required at the defined location. N+ is defined as the ECAP measurement electrode location. N- is defined as the ECAP reference electrode location.

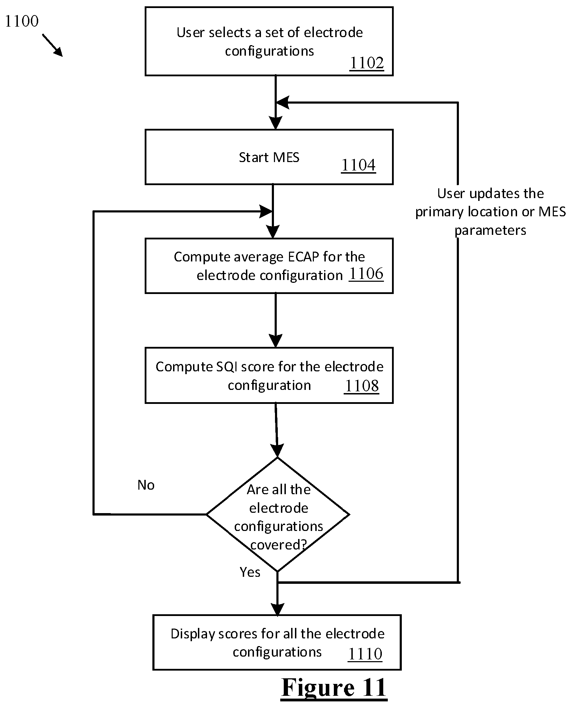

**Figure 11** is a flowchart of the MES procedure 1100 carried out by the implant. In the first step 1102, the electrode configurations are assigned to the MES program. This can be predetermined, or user determined. At 1104 the implant firmware then captures ECAP measurements of all the electrode configurations associated with the user selected setting. In an exemplary implementation, the considerations while choosing the electrodes may be that the selected stimulation, measurement, and reference electrodes must be on the same lead. Or, that only 1 stimulation electrode is selected. In this embodiment the measurement electrode must not be adjacent to the stimulation electrode, and the reference electrode must not be on the case of the implantable pulse generator, although this may be allowed in other embodiments. The measurement electrode must be between the stimulation and reference electrodes. In some cases, the primary location set by the user may not be the best location for capturing good quality ECAP recordings. The MES program will then suggest the best electrode configuration for getting a robust ECAP.

**Fig 12** shows the examples of the MES position configuration methods when the stim electrode is E2. The MES program is configured to measure ECAPs at each of the selected electrode locations until a set number of ECAPs are accumulated. Thereafter, an SQI score is calculated using the SQI algorithm at each electrode location. The SQI scores are computed for different electrode locations by the programming software, based on strategies such as fixed distance, and fixed reference, as shown in Fig 12. The MES program stops upon user intervention or after computing the score for all the selected electrodes. The user is provided with the ECAP quality score at multiple electrodes which allows the user to select the best possible electrode combination which captures the best quality ECAPs.

Example outputs are shown in Figs 13-16. **Fig 13** depicts the measurement electrode scan GUI window showing four fixed-distance recording electrode configurations' SQI. It can be determined by simple observation that E3 referenced to E7 is the best recording electrode in this example. **Figure 14** illustrates the output when the MES scan is stopped for any reason.

**Fig 15** shows the output SQI = 0% which is produced when no ECAP has been detected. These results would suggest that E7 is a poor choice of recording electrode as no ECAP is observed irrespective of reference electrode selection. **Fig. 16** illustrates the MES output when investigating which reference electrode is optimal when using E4 is the recording electrode.

It will be appreciated by persons skilled in the art that numerous variations and/or modifications may be made to the invention as shown in the specific embodiments without departing from the scope of the invention as defined by the appended claims. The present embodiments are, therefore, to be considered in all respects as illustrative and not limiting or restrictive.

## Claims

1. A system (100) for automated assessment of neural response recordings, the system (100) comprising:
a memory (118) storing a set of basis functions comprising at least one of (a) a compound action potential basis function and (b) an artefact basis function;
an input for receiving a plurality of neural recordings of electrical activity in neural tissue, the neural recordings being obtained by repeated application of stimuli using a single configuration of stimulation and recording; and
a processor configured to decompose each neural recording by determining at least one parameter which estimates at least one of a compound action potential and an artefact from the set of basis functions, the processor further configured to repeatedly determine a plurality of values of the at least one parameter for each respective one of the plurality of neural recordings; and
the processor further configured to determine a spread of the plurality of values, and the processor further configured to output an indication that the neural response recordings are of higher quality if the spread is small and the processor further configured to output an indication that the neural response recordings are of lower quality if the spread is large.

2. The system (100) of claim 1 wherein the indication of the quality of the neural response recordings is a binary indication of either high quality or low quality.

3. The system (100) of claim 1 wherein the indication of the quality of the neural response recordings is defined on a continuum, from high quality to low quality.

4. The system (100) of any one of claims 1 to 3 wherein the indication of the quality of the neural response recordings is calibrated by reference to clinician scoring of a test set of neural recordings.

5. The system (100) of any one of claims 1 to 4 wherein the processor is further configured to:
output a distinct indication of the quality of neural response recordings obtained in relation to one or more other configurations of stimulation and recording; and
select a configuration of stimulation and recording for ongoing therapy by comparing quality scores for each configuration.

6. The system (100) of any one of claims 1 to 5 wherein the spread is calculated as being one of the group consisting of:
the standard deviation of the parameters;
the variance of the parameters;
the inter-quartile range of the parameters; and
the inter-decile range of the parameters.

7. The system (100) of any one of claims 1 to 6 wherein the at least one parameter comprises at least one of the group consisting of:
a correlation of an observed ECAP with a predefined basis function comprising an analytically defined compound action potential basis function;
a frequency of an observed ECAP; and
a time offset of an observed ECAP relative to a time of a stimulus.

8. The system (100) of any one of claims 1 to 7 wherein the basis function comprises an analytically defined compound action potential basis function, and wherein the processor is further configured to use a rate at which an ECAP is detected in the plurality of recordings to define a quality of the neural response recordings.

9. The system (100) of any one of claims 1 to 8 wherein the processor is further configured to obtain two or more neural recordings of each ECAP, and to use one or more comparative parameters derived from a comparison of the two or more recordings to assess ECAP quality.

10. The system (100) of claim 9 wherein the comparative parameters comprise a conduction velocity of each ECAP determined from two or more neural recordings of that ECAP, and wherein a spread of the conduction velocity is used to derive ECAP signal quality.

11. The system (100) of any one of claims 1 to 10 wherein more than one parameter is obtained, and wherein the plurality of parameters are processed by a predefined function to generate a single quality score.

12. The system (100) of claim 11 wherein the quality score is determined as follows:$Score = \left(Detection Rate∗Correlation\right) / \left(Frequency spread + Offset spread\right) .$

13. The system of any one of claims 1 to 12 wherein the processor is further configured to normalise an ECAP signal quality score to a range [0 :1].

## Patentansprüche

1. System (100) zur automatisierten Auswertung von Aufzeichnungen neuronaler Reaktionen, wobei das System (100) Folgendes aufweist:
einen Speicher (118), der einen Satz von Basisfunktionen speichert, der mindestens eine von (a) einer Summenaktionspotenzial-Basisfunktion und (b) einer Artefakt-Basisfunktion umfasst;
einen Eingang zum Empfangen einer Mehrzahl neuronaler Aufzeichnungen elektrischer Aktivität in Nervengewebe, wobei die neuronalen Aufzeichnungen durch wiederholte Anwendung von Reizen mittels einer einzigen Konfiguration von Stimulation und Aufzeichnung erzielt werden; und
einen Prozessor, der dazu ausgebildet ist, jede neuronale Aufzeichnung zu zerlegen, indem er mindestens einen Parameter bestimmt, der mindestens eines von einem Summenaktionspotenzial und einem Artefakt aus dem Satz von Basisfunktionen schätzt,
wobei der Prozessor ferner dazu ausgebildet ist, wiederholt eine Mehrzahl von Werten des mindestens einen Parameters für jede einzelne der Mehrzahl neuronaler Aufzeichnungen zu bestimmen; und
wobei der Prozessor ferner dazu ausgebildet ist, eine Streuung der Mehrzahl von Werten zu bestimmen, und wobei der Prozessor ferner dazu ausgebildet ist, eine Angabe dahingehend auszugeben, dass die Aufzeichnungen neuronaler Reaktionen von höherer Qualität sind, wenn die Streuung gering ist, und der Prozessor ferner dazu ausgebildet ist, eine Angabe dahingehend auszugeben, dass die Aufzeichnungen neuronaler Reaktionen von geringerer Qualität sind, wenn die Streuung groß ist.

2. System (100) nach Anspruch 1,
wobei die Angabe der Qualität der Aufzeichnungen neuronaler Reaktionen eine binäre Angabe von entweder hoher Qualität oder niedriger Qualität ist.

3. System (100) nach Anspruch 1,
wobei die Angabe der Qualität der Aufzeichnungen neuronaler Reaktionen auf einem Kontinuum von hoher Qualität bis niedrige Qualität definiert ist.

4. System (100) nach einem der Ansprüche 1 bis 3,
wobei die Angabe der Qualität der Aufzeichnungen neuronaler Reaktionen durch Bezugnahme auf die klinische Bewertung eines Testsatzes neuronaler Aufzeichnungen kalibriert wird.

5. System (100) nach einem der Ansprüche 1 bis 4,
wobei der Prozessor ferner dazu ausgebildet ist, eine eindeutige Angabe der Qualität der Aufzeichnungen neuronaler Reaktionen auszugeben, die in Relation zu einer oder mehreren anderen Konfigurationen von Stimulation und Aufzeichnung erhalten werden, sowie eine Konfiguration von Stimulation und Aufzeichnung für die laufende Therapie durch Vergleich von Qualitätswerten für jede Konfiguration auszuwählen.

6. System (100) nach einem der Ansprüche 1 bis 5,
wobei die Streuung berechnet wird als eine aus der Gruppe bestehend aus:
der Standardabweichung der Parameter;
der Varianz der Parameter;
dem Interquartilsabstand der Parameter; und
dem Interdezilbereich der Parameter.

7. System (100) nach einem der Ansprüche 1 bis 6,
wobei der mindestens eine Parameter mindestens einen umfasst aus der Gruppe bestehend aus:
einer Korrelation eines beobachteten ECAP mit einer vordefinierten Basisfunktion, die eine analytisch definierte Summenaktionspotenzial-Basisfunktion umfasst;
einer Frequenz eines beobachteten ECAP; und
einer Zeitverschiebung eines beobachteten ECAP relativ zu einem Zeitpunkt eines Reizes.

8. System (100) nach einem der Ansprüche 1 bis 7,
wobei die Basisfunktion eine analytisch definierte Summenaktionspotenzial-Basisfunktion umfasst und wobei der Prozessor ferner dazu ausgebildet ist, eine Rate, mit der ein ECAP in der Mehrzahl von Aufzeichnungen erkannt wird, zum Definieren einer Qualität der Aufzeichnungen neuronaler Reaktionen zu verwenden.

9. System (100) nach einem der Ansprüche 1 bis 8,
wobei der Prozessor ferner dazu ausgebildet ist, zwei oder mehr neuronale Aufzeichnungen jedes ECAP zu ermitteln und einen oder mehrere Vergleichsparameter zu verwenden, die aus einem Vergleich der zwei oder mehr Aufzeichnungen abgeleitet werden, um die ECAP-Qualität zu beurteilen.

10. System (100) nach Anspruch 9,
wobei die Vergleichsparameter eine Leitungsgeschwindigkeit jedes ECAP umfassen, die aus zwei oder mehr neuronalen Aufzeichnungen dieses ECAP bestimmt wird, und wobei eine Streuung der Leitungsgeschwindigkeit zur Ableitung der ECAP-Signalqualität verwendet wird.

11. System (100) nach einem der Ansprüche 1 bis 10,
bei dem mehr als ein Parameter ermittelt wird, und wobei die Mehrzahl der Parameter mittels einer vordefinierten Funktion verarbeitet wird, um einen einzigen Qualitätswert zu generieren.

12. System (100) nach Anspruch 11,
wobei die Qualitätsbewertung wie folgt bestimmt wird:$Ergebnis = \left(Erkennungsrate ∗ Korrelation\right) / \left(Frequenzstreuung + Offsetstreu-ung\right) .$

13. System nach einem der Ansprüche 1 bis 12,
wobei der Prozessor ferner dazu ausgebildet ist, einen ECAP-Signalqualitätswert auf einen Bereich [0: 1] zu normalisieren.

## Revendications

1. Système (100) destiné à l'évaluation automatisée d'enregistrements de réponse neuronale, le système (100) comprenant :
une mémoire (118) stockant un ensemble de fonctions de base comprenant au moins l'une parmi (a) une fonction de base de potentiel d'action de composé et (b) une fonction de base d'artefact ;
une entrée destinée à recevoir une pluralité d'enregistrements neuronaux de l'activité électrique dans le tissu neuronal, les enregistrements neuronaux étant obtenus par l'application répétée de stimuli à l'aide d'une configuration unique de stimulation et d'enregistrement ; et
un processeur configuré pour décomposer chaque enregistrement neuronal en déterminant au moins un paramètre qui estime au moins l'un parmi un potentiel d'action de composé et un artefact à partir de l'ensemble de fonctions de base, le processeur étant en outre configuré pour déterminer de manière répétée une pluralité de valeurs de l'au moins un paramètre pour chaque enregistrement respectif de la pluralité d'enregistrements neuronaux ; et
le processeur étant en outre configuré pour déterminer une dispersion de la pluralité de valeurs, et le processeur étant en outre configuré pour délivrer en sortie une indication selon laquelle les enregistrements de réponse neuronale sont de qualité supérieure si la dispersion est faible, et le processeur étant en outre configuré pour délivrer en sortie une indication selon laquelle les enregistrements de réponse neuronale sont de qualité inférieure si la dispersion est grande.

2. Système (100) selon la revendication 1, dans lequel l'indication de la qualité des enregistrements de réponse neuronale est une indication binaire indiquant soit une qualité élevée, soit une qualité faible.

3. Système (100) selon la revendication 1, dans lequel l'indication de la qualité des enregistrements de réponse neuronale est définie sur un continuum allant de qualité élevée à qualité faible.

4. Système (100) selon l'une quelconque des revendications 1 à 3, dans lequel l'indication de la qualité des enregistrements de réponse neuronale est étalonnée par référence à la notation par un clinicien d'un ensemble de test d'enregistrements neuronaux.

5. Système (100) selon l'une quelconque des revendications 1 à 4, dans lequel le processeur est en outre configuré pour :
délivrer en sortie une indication distincte de la qualité des enregistrements de réponse neuronale obtenus en relation avec une ou plusieurs autres configurations de stimulation et d'enregistrement, et
sélectionner une configuration de stimulation et d'enregistrement pour la thérapie en cours en comparant les scores de qualité pour chaque configuration.

6. Système (100) selon l'une quelconque des revendications 1 à 5, dans lequel la dispersion est calculée comme étant l'un parmi le groupe consistant en :
l'écart-type des paramètres ;
la variance des paramètres ;
l'intervalle interquartile des paramètres ;
et l'intervalle interdécile des paramètres.

7. Système (100) selon l'une quelconque des revendications 1 à 6, dans lequel l'au moins un paramètre consiste en au moins l'un parmi le groupe consistant en :
une corrélation entre un ECAP (Evoked Compound Action Potential pour « potentiel d'action de composé évoqué ») observé et une fonction de base prédéfinie comprenant une fonction de base de potentiel d'action de composé définie analytiquement,
une fréquence d'un ECAP observé ; et
un décalage temporel d'un ECAP observé par rapport au moment d'un stimulus.

8. Système (100) selon l'une quelconque des revendications 1 à 7, dans lequel la fonction de base comprend une fonction de base de potentiel d'action de composé définie analytiquement, et dans lequel le processeur est en outre configuré pour utiliser un taux auquel un ECAP est détecté dans la pluralité d'enregistrements pour définir une qualité des enregistrements de réponse neuronale.

9. Système (100) selon l'une quelconque des revendications 1 à 8, dans lequel le processeur est en outre configuré pour obtenir deux ou plus de deux enregistrements neuronaux de chaque ECAP, et pour utiliser un ou plusieurs paramètres comparatifs dérivés d'une comparaison des deux ou plus de deux enregistrements pour évaluer la qualité de l'ECAP.

10. Système (100) selon la revendication 9, dans lequel les paramètres comparatifs comprennent une vitesse de conduction de chaque ECAP déterminée à partir de deux ou plus de deux enregistrements neuronaux de cet ECAP, et dans lequel une dispersion de la vitesse de conduction est utilisée pour dériver la qualité du signal ECAP.

11. Système (100) selon l'une quelconque des revendications 1 à 10, dans lequel plus d'un paramètre est obtenu, et dans lequel la pluralité de paramètres est traitée par une fonction prédéfinie pour générer un score de qualité unique.

12. Système (100) selon la revendication 11, dans lequel le score de qualité est déterminé comme suit :$Score = \left(Taux de detection ∗ Correlation\right) / \left(Ecart de frequence + Ecart de decalage\right) .$

13. Système selon l'une quelconque des revendications 1 à 12, dans lequel le processeur est en outre configuré pour normaliser un score de qualité du signal ECAP sur une plage [0 :1].
